Europäisches Patentamt

(19) European Patent Office

Office européen des brevets

(11) Numéro de publication : **0 340 288 B1**

**FASCICULE DE BREVET EUROPEEN**

(45) Date de publication du fascicule du brevet :
**14.04.93 Bulletin 93/15**

(51) Int. Cl.⁵ : **A61B 5/04**, A61N 1/05

(21) Numéro de dépôt : **88910052.5**

(22) Date de dépôt : **27.10.88**

(86) Numéro de dépôt international :
**PCT/FR88/00525**

(87) Numéro de publication internationale :
**WO 89/03657 05.05.89 Gazette 89/10**

(54) **PROCEDE ET SONDE AZIMUTALE POUR REPERER LE POINT D'EMERGENCE DES TACHYCARDIES VENTRICULAIRES.**

(30) Priorité : **27.10.87 FR 8715027**

(43) Date de publication de la demande :
**08.11.89 Bulletin 89/45**

(45) Mention de la délivrance du brevet :
**14.04.93 Bulletin 93/15**

(84) Etats contractants désignés :
**AT BE CH DE GB IT LI LU NL SE**

(56) Documents cités :
**FR-A- 2 561 929**
**FR-A- 2 569 103**

(56) Documents cités :
**IEEE 1985 COMPINT - Computer Aided Technologies, Montreal, Quebec, Canada, 9-13 September 1985, IEEE, (US), P. Savard et al.: "Interactive electrophysiologic mapping system for on-line analysis of cardiac activation sequences", pages 76-78**
**Journal of the American College of Cardiology, Vol. 2, No. 5, November 1983, The American College of Cardiology, J.M.T. de Bakker et al.: "Endocardial mapping by simultaneous recording of endocardial electrograms during cardiac surgery for ventricular aneurysm", pages 947-953**

(73) Titulaire : **GLACE, Christian**
**12, rue du Vivarais**
**F-54500 Vandoeuvre (FR)**

(72) Inventeur : **GLACE, Christian**
**12, rue du Vivarais**
**F-54500 Vandoeuvre (FR)**

(74) Mandataire : **Poupon, Michel**
**B.P. 421 3, rue Ferdinand Brunot**
**F-88011 Epinal Cédex (FR)**

EP 0 340 288 B1

Jouve, 18, rue Saint-Denis, 75001 PARIS

## Description

La présente invention concerne un dispositif permettant le repérage du point d'émergence des tachycardies ventriculaires en médecine cardiologique, par une méthode d'approches successives, caractérisée, comparativement à la cartographie cardiaque traditionnelle, par : une précision du même ordre, sa rapidité de mise en oeuvre, son faible coût, et la possibilité supplémentaire de repérer le trajet des macro-réentrées (autour des anévrysmes par exemple).

Contrairement à la cartographie traditionnelle, ce dispositif dispense de la construction d'une carte d'isochrones.

Les techniques actuellement utilisées sont soit manuelles, soit automatiques et s'appliquent à l'épicarde, à l'endocarde, ou au myocarde lui-même :

A) Epicarde/Méthode manuelle : une sonde tripolaire est déplacée à la surface du coeur, alors qu'une électrode de référence est accrochée près de la base de l'aorte.

Un électrocardiographe comportant :

- une trace pour l'ECG de surface,
- deux traces pour les électrogrammes de la référence et de la sonde mobile,

permet de mesurer la distance horizontale sur le papier entre les déflections,au temps écoulé (délai) entre les dépolarisations perçues par l'électrode de référence et l'électrode mobile.

Plusieurs délais permettent la construction d'une carte d'isochrones (courbes d'égaux délais) donnant une vue bidimensionnelle de la propagation de la dépolarisation. Le point de cette carte correspondant au délai le plus court, est considéré comme étant le point d'émergence, qui fait donc l'objet d'une élimination chirurgicale. Cette élimination peut se faire par ventriculotomie au bistouri, par diathermie, ou par technique cryochirurgicale.

B) Epicarde/Méthode automatique : un filet épicardique supporte de 50 à plus de 300 électrodes reliées chacune à un amplificateur d'instrumentation. Chaque voie est multiplexée, échantillonnée-bloquée et convertie en numérique. Un processeur acquiert ces données, les traite et les visualise sur un moniteur ou une imprimante, sous forme de liste ou d'image.

C) Myocarde : une aiguille munie de plusieurs électrodes renseigne sur la profondeur du point d'émergence dans l'épaisseur du myocarde.

D) Endocarde/Méthode manuelle: un cathéter est positionné en différents points de l'endocarde. Le lieu correspondant au délai le plus précoce, par rapport au cathéter de référence, fait l'objet d'une fulguration.

E) Endocarde/Méthode automatique : un ballon gonflable endocavitaire comportant plusieurs dizaines d'électrodes permet, à l'aide du système d'acquisition décrit en B), la localisation du point d'émergence.

Le principe du dispositif selon l'invention est basé sur le repérage de la propagation de la dépolarisation cellulaire myocardique, ou de la contraction musculaire du coeur qui en résulte directement. Ce repérage se fait par étapes, tout au long de son trajet, à partir d'un point arbitraire sur le coeur choisi par l'opérateur, en remontant ce trajet jusqu'à son origine, qui est dénommée le point d'émergence.

Le dispositif selon l'invention comprend une sonde reliée à des circuits électroniques destinés à traiter les signaux recueillis par la sonde et à commander un interface dispositif opérateur.Le dispositif selon l'invention est dénommé : "sonde -azimutale", les deux mots n'étant pas dissociés dans ce cas. Le terme de "sonde" est réservé à un ensemble comportant les capteurs, leur support, le câble électrique de liaison avec son connecteur, et d'éventuels moyens de signalisation qui y sont fixés et destinés à l'opérateur.

La sonde est composée d'au moins un ensemble de deux capteurs sensibles à la dépolarisation myocardique (phénomène électrochimique), ou à la contraction cardiaque (phénomène mécanique qui en est la conséquence). Chaque ensemble de deux capteurs est disposé sur un axe approximativement rectiligne. Les capteurs sont fixés sur un support que l'opérateur déplace sur l'épicarde ou sur l'endocarde.

On connait déjà des dispositifs, par exemple celui du document FR-A-2 569 103 qui comportent des ensembles de deux capteurs disposés rectilignement, mais les signaux produits par les capteurs sont enregistrés séparément pour construire des "cartes cardiaques", alors que dans l'invention les signaux produits sont utilisés pour contruire un signal de déphasage.

Dans ce but, la sonde azimutale permet de déterminer :

- le sens de la propagation de la dépolarisation cellulaire myocardique ou de la contraction musculaire cardiaque qui en résulte directement, dont l'origine commune est l'activation du point d'émergence ; ce sens est déterminé sur la direction représentée par l'axe des deux capteurs, lorsque l'opérateur peut noter l'ordre d'arrivée des potentiels d'activation sous l'un, puis sous l'autre capteur (déphasage) ; si ces arrivées sont simultanées, le déphasage est nul.
- la direction de cette propagation par plusieurs mesures de déphasage comme ci-dessus, lors de la rotation de la sonde autour d'un axe, passant entre les deux capteurs, et perpendiculaire à la surface cardiaque ; la direction est déterminée, soit lorsque l'opérateur peut noter le déphasage maximum, l'axe des capteurs étant alors confondu avec celle-ci, soit lorsque l'opérateur peut noter un déphasage nul, l'axe des capteurs étant alors perpendiculaire à celle-ci.

- le trajet de la dépolarisation, en partant d'un point arbitraire du coeur, et en réalisant à chaque étape ; une détermination de sens et de direction telles qu'elles ont été décrites ci-dessus, puis un déplacement de la sonde sur ou dans le coeur d'une distance de l'ordre du centimètre, à déterminer à l'usage, dans le sens opposé et dans la direction qui viennent d'être déterminés.

Le point d'émergence est atteint après plusieurs étapes telles qu'elles viennent d'être décrites. Ce point est reconnu par le fait que la dépolarisation se propage de façon centripète à partir de lui. Les déphasages mesurés à ce point sont donc tous nuls.

Dans le but d'améliorer la discrimination d'azimut lors d'une même mesure, les ensembles de deux capteurs sont associés sur une même sonde suivant deux ou plusieurs directions. Chaque ensemble de capteurs fournit des informations qui sont transmises à l'opérateur. L'incertitude $\varepsilon$ sur la direction qui se calcule par :

$$\varepsilon = \frac{180}{n} \qquad \text{où :} \quad \varepsilon \text{ est exprimé en degrés}$$
$$n = \text{nombre de groupes de deux capteurs}$$

Pour éviter la multiplication des capteurs, le dispositif ne comporte, dans une version améliorée, que deux ensembles de deux capteurs disposés suivant deux axes perpendiculaires. Chaque ensemble fournit deux signaux combinés en un signal de déphasage. Chaque déphasage, reporté sur un repère orthonormé, est considéré comme étant la projection d'un vecteur sur ce repère. Ce vecteur se confond approximativement avec l'azimut de la dépolarisation. Le vecteur désigne le sens et la direction dans lesquels il faut se déplacer pour réaliser la prochaine étape de mesure.

De façon accessoire, l'opérateur peut ensuite repérer la profondeur du foyer d'activation de la tachycardie en enfonçant, par étapes successives, une aiguille comportant un ensemble de deux capteurs, au lieu déterminé précédemment, à savoir le point d'émergence. Ce point est en fait la projection électrique du foyer d'activation, sur l'endocarde ou l'épicarde selon la voie d'abord qu'utilise l'opérateur. Le foyer d'activation est trouvé lorsque le déphasage entre les deux signaux relevés par les capteurs est nul (pas de sens préférentiel). La direction n'a pas besoin d'être déterminée par le dispositif puisqu'elle est représentée par l'axe de l'aiguille. Dans un second temps, l'opérateur utilise un moyen de destruction de la zone ainsi repérée, dont le fonctionnement est autorisé par le dispositif et dont la partie active est située sur l'aiguille. A titre d'exemple, cette partie active peut être :

- un groupe d'électrodes véhiculant un courant électrique produit par un générateur de diathermie haute fréquence (bistouri électrique) ou un défibrillateur cardiaque,
- une résistance chauffante,
- un canal transportant un fluide réfrigérant (cryochirurgie).

Le dispositif selon l'invention comporte des circuits électroniques destinés : à traiter le signal fourni par les capteurs, à fournir des signaux porteurs des informations recherchées (azimut de la dépolarisation et lieu du point d'émergence), à commander éventuellement un dispositif de destruction interne ou fourni par l'utilisateur. Chaque capteur est suivi d'un circuit de traitement comportant : un amplificateur, un filtre destiné à sélectionner la composante utile du signal, et de comparateurs à hystérésis transformant les signaux analogiques en signaux logiques binaires. Ce circuit est dénommé : "canal". Les canaux sont groupés par deux, correspondant aux ensembles de deux capteurs. Deux canaux correspondants sont connectés en sortie à un détecteur de phase logique, dont le signal résultant est caractéristique du déphasage entre les signaux fournis par l'ensemble de deux capteurs considéré. Dans le cas du procédé vectoriel décrit ci-dessus, les deux déphasages issus des quatre capteurs, sont combinés de façon à reconstruire le vecteur recherché.

Le dispositif est conçu de telle façon que le courant de fuite vers le patient soit maintenu dans les limites légales (par exemple : alimentation exclusive par accumulateurs, utilisation d'amplificateurs d'isolation ou de coupleurs optiques).

Chaque amplificateur est protégé, par un limiteur, des surtensions éventuellement produites par des appareillages utilisés simultanément par l'opérateur.

Lorsqu'une tachycardie ventriculaire n'est pas spontanée, il est nécessaire de l'induire par un générateur d'impulsions appelé "stimulateur cardiaque externe", relié au coeur par des conducteurs-électrodes. Afin de conserver sa fonctionalité au présent dispositif, il faut insérer un interrupteur analogique dans la chaîne de mesure, commandé par les impulsions de stimulateur.

Tous les signaux issus des circuits décrits ci-dessus sont acheminés vers un moyen d'interface entre le dispositif et l'opérateur, par l'intermédiaire de l'un de ses sens physiologiques : vue, ouïe, toucher, lui indiquant :

- d'une part, soit le sens et la direction dans lesquels se propage la dépolarisation myocardique, soit le sens et la direction dans lesquels il faut déplacer la sonde pour réaliser la prochaine étape d'acquisition.
- d'autre part, le cas échéant, l'absence de déphasage dans toutes les directions, et donc, la localisation du point d'émergence sous l'emplacement actuel de la sonde ; ce résultat donne l'autorisation d'utiliser le moyen de destruction intégré dans la sonde azimutale, ou toute autre dispositif externe fourni par l'utilisateur.

Les fonctions réalisées par les circuits électriques cablés décrits ci-dessus, sont réalisés, dans une autre version du dispositif, par un calculateur recevant les signaux issus des capteurs après amplification, et réalisant des fonctions similaires caractérisées en ce qu'elles sont programmées. Ce calculateur est pourvu de ses périphériques de conversion analogique-numérique et d'interface avec l'utilisateur.

La figure 1 représente des réalisations, données à titre d'exemple, de l'extrémité de la sonde dans la version de base du dispositif selon l'invention ; en "a" la sonde épicardique, en "b" le cathéter endocardique.

La figure 2 représente, en coupe, la paroi du coeur, contenant le foyer d'activation et les points d'émergence correspondants.

La figure 3 représente en "a", la propagation de la dépolarisation cardiaque à partir du point d'émergence, et en "b" un trajet de l'onde de dépolarisation avec deux étapes de mesure réalisées avec le dispositif.

La figure 4 représente des réalisations, données à titre d'exemple, de l'extrémité de la sonde dans des versions améliorées.

La figure 5 représente les quatre signaux issus de la version vectorielle du dispositif, groupés par deux, produisant après traitement les deux valeurs de déphasage.

La figure 6 représente la reconstitution du vecteur de dépolarisation à l'aide des deux précédents déphasages.

La figure 7 représente ce vecteur en superposition avec la position actuelle de la sonde et les directions et sens dans lesquels l'opérateur doit déplacer la sonde pour réaliser la prochaine étape d'acquisition.

La figure 8 représente le schéma bloc des circuits électroniques de base utilisés dans le dispositif.

La figure 9 représente le schéma bloc du moyen d'élimination des impulsions provenant du stimulateur cardiaque externe.

La figure 10 représente le schéma bloc de l'interface dispositif-opérateur.

La figure 11 représente le schéma-bloc de la version programmée du dispositif.

La figure 12 représente le limiteur destiné à protéger l'amplificateur des surtensions.

La figure 13 représente, en coupe, une réalisation de l'aiguille-sonde donnée à titre d'exemple, avec la disposition des électrodes, de la partie active du moyen de destruction situé sur l'aiguille-sonde et du générateur qui y est associé.

La figure 14a représente une réalisation, donnée à titre d'exemple, de l'interface dispositif-utilisateur, précédé du détecteur qui lui correspond. La figure 14b représente la disposition des voyants lumineux sur la sonde, dont la commande est effectuée par le circuit de la figure 14a.

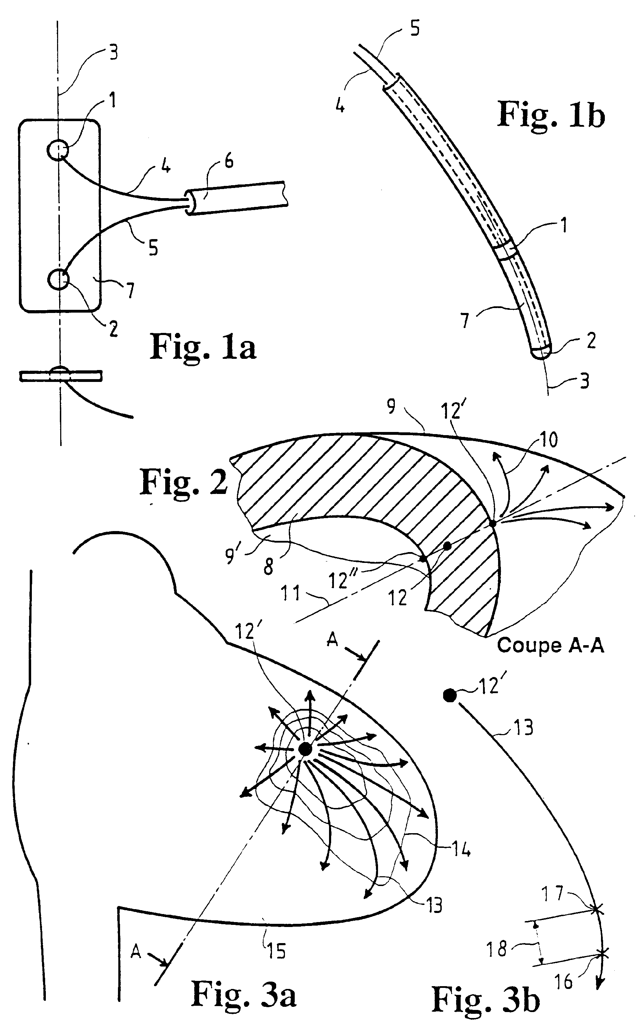

Sur la figure 1, les exemples de sonde comportent deux capteurs (1) et (2), disposés sur un axe approximativement rectiligne (3), fixés sur un support (7), et munis de conducteurs électriques (4) et (5), eux-mêmes contenus dans un câble (6) qui véhicule les signaux destinés aux circuits électroniques du dispositif.

La figure 2 représente, dans une portion de myocarde (8) en coupe, le foyer d'activation (12) de la tachycardie ventriculaire, et ses projections électriques (12′) et (12″) sur l'épicarde (9) et sur l'endocarde (9′), c'est-à-dire les points d'émergence. La ligne (10) représente un exemple de trajet suivi par la dépolarisation myocardique à partir du foyer d'activation (12).

La figure 3a montre un coeur (15), comportant un point d'émergence épicardique (12′), produisant des dépolarisations centripètes (13), dont les vitesses sont variables en fonction des conditions locales de conduction. Les courbes d'égaux délais de dépolarisation à partir du point (12′), sont nommées isochrones (14).

La figure 3b représente un détail de la figure 3a, situant un point de départ arbitraire (16) lors de l'utilisation du dispositif selon l'invention, ainsi que la première étape (17), située à une distance (18) du point de départ. La distance (18) est fonction de l'expérience de l'opérateur. Elle sera plus réduite (quelques millimètres) pour un débutant. L'orientation du point (17) par rapport à (16) est déterminé en fonction des indications du dispositif lorsqu'il se trouve en (16).

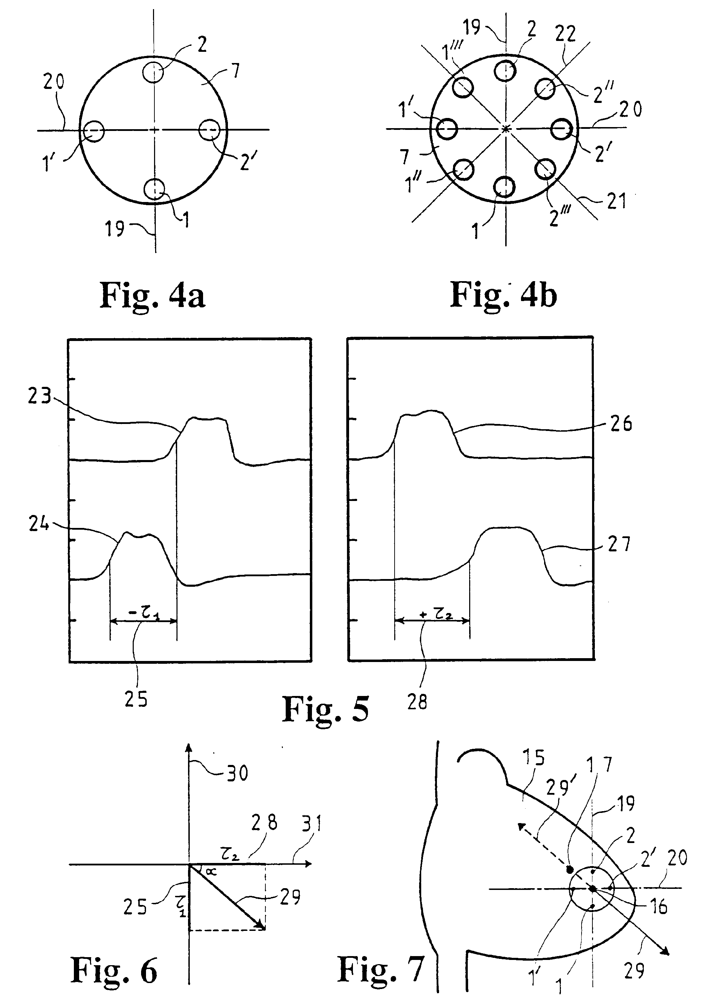

La description qui suit donne un exemple d'une réalisation du dispositif selon l'invention. L'extrémité utile de la sonde est représentée sur la figure 4. Deux ensembles de deux capteurs (1), (2) et (1′), (2′) sont disposés orthogonalement selon deux axes (19) et (20), sur un support (7). Les capteurs sont distants de moins de 50 millimètres. Cette distance est inversement proportionnelle à la capacité des circuits à discriminer le déphasage des signaux, fonction de l'encombrement toléré pour la sonde et proportionnel à la précision recherchée pour le dispositif. Chaque capteur est, dans le cas présent, composé de deux électrodes reliées chacune à une entrée de l'amplificateur d'instrumentation dont l'impédance d'entrée est réglable. L'écartement des électrodes et l'impédance doivent être suffisamment faibles pour séparer les potentiels locaux des potentiels lointains générés par le myocarde, sachant que des valeurs trop faibles d'écartement et d'impédance ne permettent plus de relever des signaux d'amplitude suffisante.

Quelques essais de sondes de différentes dimensions permettent de trouver le meilleur compromis.

Les signaux (23), (24) et (26), (27) issus respectivement des capteurs (1), (2) et (1'), (2') et représentées sur la figure 5 sont comparés, deux à deux, du point de vue de leur phase, résultant en des signaux de déphasage (25) et (28).

En figure 6, les valeurs algébriques de ces déphasages sont reportées sur un repère (30) et (31) orthonormé. Ces deux segments représentent les projections dans le plan du support (7) de la sonde, d'un vecteur (29) superposable à la direction et au sens de la dépolarisation myocardique. L'angle trigonométrique $\alpha$ fait avec le demi-axe positif de (29), se calcule par :

$$\alpha = \text{Arc tg} \ \frac{\tau_1}{\tau_2} \qquad \text{où} : \tau_1 = \text{retard} \ (23)$$
$$\tau_2 = \text{retard} \ (26)$$
$$\text{et} : (\tau_1 , \tau_2) \in R^2$$

L'angle $\alpha$ est dit "azimut" de la dépolarisation au lieu où se trouve la sonde.

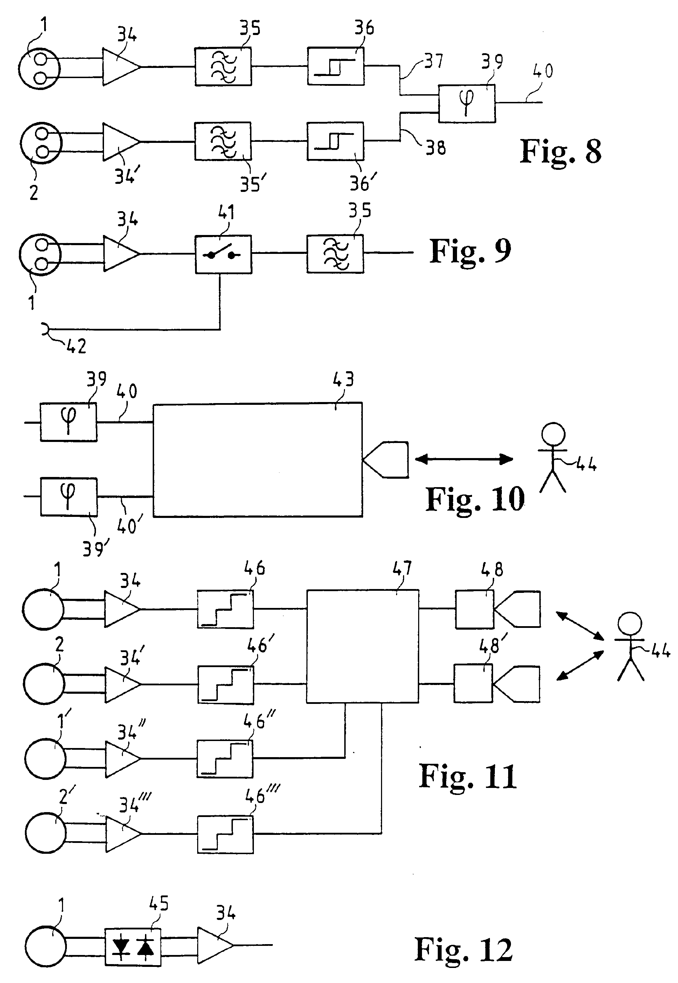

Sur la figure 8, les signaux sont traités par des circuits électroniques analogiques et numériques cablés comprenant, par capteur (1) ou (2) :

- un amplificateur (34) ou (34') qui amène le signal à une tension et une impédance compatible avec les circuits suivants,
- un filtre (35) ou (35') destiné à sélectionner la portion du signal utile,
- un comparateur à hystérésis (36) ou (36') qui met en forme le signal afin qu'il puisse être exploité par des circuits logiques.

Les deux sorties résultantes (37) et (38) sont comparées par un détecteur de phase (39) produisant un signal (40) caractéristique du déphasage entre les événements captés par (1) et (2). Les deux lignes (37) et (38) véhiculent respectivement les signaux (23) et (24) de la figure 5 ; la ligne (40) véhicule une impulsion qui correspond au retard (25) sur la figure 5 ; ce retard a une valeur mesurée en millisecondes et a un signe algébrique suivant que (24) est en avance ou en retard sur (23).

Le circuit représenté à la figure 8 doit être dédoublé pour traiter les signaux issus des capteurs (1') et (2') de la figure 4a. Deux signaux de déphasage (40) et (40') en résultent.

Sur la figure 10, l'indication d'azimut est fourni à l'opérateur (44) par un moyen d'interface (43) collectant les signaux de déphasage (40) et (40') issus des deux comparateurs de phase (39) et (39').

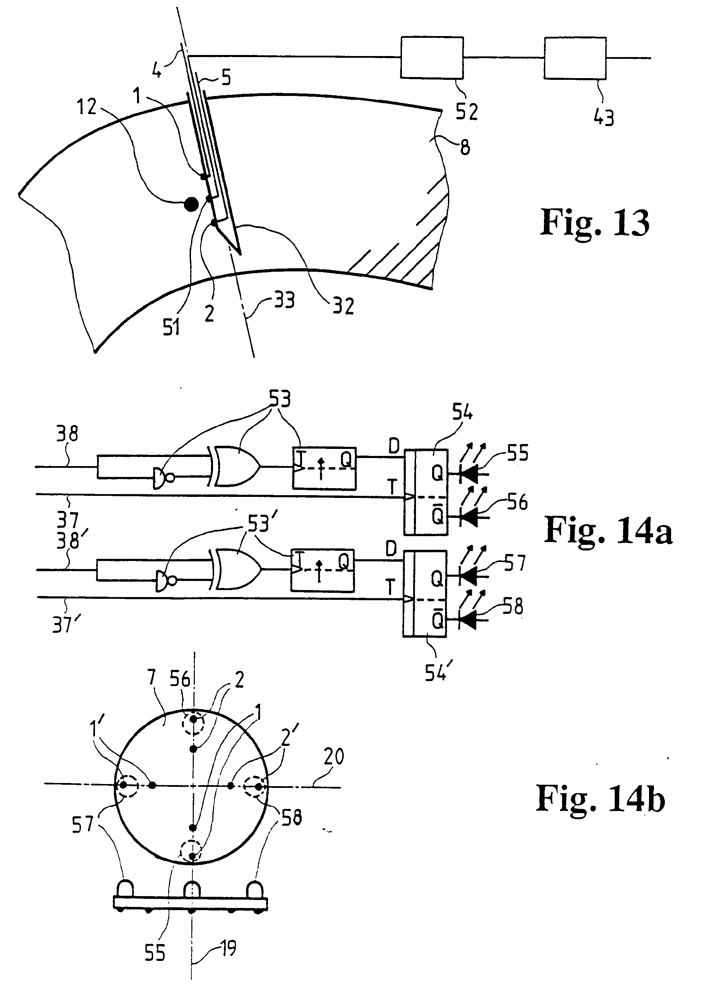

Un exemple simple de réalisation de l'interface est donné en figure 14a. Les signaux (38), via un monostable (53) réglé à 50ms déclenché par les fronts montants et descendants, et (37) commandent une bascule D dont les sorties complémentaires Q et Q se positionnent à un niveau logique 0 ou 1 en fonction de l'ordre d'apparition des signaux à l'entrée. Deux parmi les quatre diodes électroluminescentes (55), (56), (57) et (58) s'allument pour indiquer l'azimut d'où provient la dépolarisation. L'opérateur déplace donc la sonde dans cette direction et ce sens pour effectuer la prochaine étape d'acquisition.

Sur la figure 9, s'il est utilisé, un stimulateur cardiaque externe, relié directement à l'entrée (42), sert à commander une porte analogique (41) bloquant ou non les signaux en provenance de la sonde. En l'absence d'impulsion, l'interrupteur (41) est fermé, et laisse passer les signaux provenant du capteur (1). Pendant l'impulsion, l'interrupteur (41) est ouvert et empêche le signal correspondant à l'impulsion, qui est conduite par le coeur, de perturber le fonctionnement du dispositif.

Sur la figure 12, l'amplificateur (34) est protégé en entrée, par un limiteur (45), contre les surtensions qui sont induites par les dispositifs que peut utiliser l'opérateur à proximité de la sonde (par exemple : bistouri électrique, défibrillateur).

La figure 13, représente la localisation, sur l'aiguille (32) par exemple, de la partie active (51) du moyen de destruction du foyer d'activation (12) de la tachycardie ventriculaire.

Dans une seconde version du dispositif représentée sur la figure 11, les circuits électroniques sont réalisés en technique logique programmée, réalisant des fonctions similaires à la version câblée et comportant : des capteurs (1), (2) et (1'), (2') reliés à des amplificateurs (34), (34') et (34''), (34'''), suivis de convertisseurs analogiques-numériques munis d'échantillonneurs bloqueurs (46), (46') et (46''), (46'''), et un processeur programmé (47) qui réalise les acquisitions, filtre et reconnaît les signaux, les compare et indique à l'opérateur (44) par les interfaces (48) et (48') la marche à suivre pour atteindre le point d'émergence. Le processeur (47) réalise également, le cas échéant, la suppression de l'impulsion de stimulation dans le traitement du signal. Les quatre systèmes de conversion (46), (46'), (46") et (46"') peuvent être remplacés par quatre échantillonneurs-bloqueurs, suivis d'un multiplexeur analogique, suivi d'un seul convertisseur analogique-numérique.

**Revendications**

1. Dispositif destiné à la localisation du point d'émergence des tachycardies ventriculaires en médecine cardiologique, caractérisé en ce qu'il comporte :

   a) un ensemble de deux capteurs (1) et (2), distants de moins de cinquante millimètres et disposés sur un axe rectiligne (3).

   b) un dispositif électronique de contrôle, comportant autant d'amplificateurs (34) qu'il y a de capteurs (1) ou (2), suivis chacun de filtres (35) destinés à selectionner la composante utile du signal, et de comparateurs à hystérésis (36) produisant des signaux logiques, ces signaux logiques, groupés par deux (37) et (38), ayant pour origine les ensembles de deux capteurs (1) et (2), sont comparés par un détecteur de phase logique (39) produisant un signal (40) caractéristique du déphasage entre les signaux fournis par les deux capteurs.

   c) un dispositif de signalisation (54,55,56) réalisant l'interface dispositif-opérateur.

2. Dispositif selon la revendication 1 caractérisé en ce qu'il comporte des ensembles de deux capteurs (1) et (2) disposés sur un axe et associés sur une même sonde suivant deux ou plusieurs directions (19, 20, 21 et (22).

3. Dispositif selon les revendications 1 et 2 caractérisé en ce qu'il comporte deux ensembles de deux capteurs, disposés suivant deux axes perpendiculaires (19 et 20), produisant au moyen d'un circuit deux signaux déphasés (25) et (28), images des projections dans le plan du support (7) de la sonde, d'un vecteur (29) image de la dépolarisation myocardique, et en ce qu'il comporte une interface (43) de recomposition dudit vecteur (29).

4. Dispositif selon la revendication 1 caractérisé en ce que les deux capteurs (1) et (2) sont disposés sur une aiguille (32) de localisation du foyer d'activation.

5. Dispositif selon la revendication 4 caractérisé en ce que l'aiguille comporte un moyen de destruction (51) et (52) du point d'émergence autorisé par l'interface (43) et commandé par l'opérateur.

6. Dispositif selon la revendication 1 caractérisé en ce que les amplificateurs (34) comportent un limiteur (45) de protection des surtensions.

7. Dispositif selon la revendication 1 caractérisé en ce qu'il comporte un interrupteur analogique (41) éliminant les signaux provenant d'un stimulateur cardiaque, l'interrupteur étant commandé par les impulsions électriques directement dérivées du stimulateur.

8. Dispositif selon la revendication 1 caractérisé en ce que le dispositif de signalisation comporte un moyen d'interface (43) entre le dispositif et l'opérateur, par l'intermédiaire de l'un de ses sens physiologiques : vue, ouïe, toucher, lui indiquant :
   - d'une part, soit le sens et la direction (29) dans lesquels se propage la dépolarisation myocardique, soit le sens opposé sur la même direction (29') indiquant l'orientation du déplacement de la sonde pour réaliser la prochaine étape d'acquisition.
   - d'autre part, le cas échéant, la localisation du point d'émergence (12' ou 12") sous la sonde, en l'absence de déphasage dans toutes les directions.

9. Dispositif selon les revendications précédentes prises dans leur ensemble, caractérisé en ce qu'il comporte un calculateur (47) de traitement du signal après amplification et pourvu de périphériques d'échantillonnage-blocage et de conversion analogique-numérique (46), (46'), (46") et (46"') et d'interfaçage (48) et (48) avec l'utilisateur (44).

**Patentansprüche**

1. Vorrichtung zur Ermittlung der Austrittsstelle ventriculärer Tachykardien für die Herzmedizin, **dadurch gekennzeichnet**, daß sie folgendes enthält:

a) eine Anordnung von zwei Sonden (1) und (2), welche weniger als 50 mm Abstand zueinander haben und auf einer geradlinigen Achse (3) angeordnet sind;

b) eine elektronische Steuereinrichtung, welche ebenso viele Verstärker (34) aufweist, wie sie Sonden (1) oder (2) hat, denen jeweils Filter (35) zum Selektieren der Nutzanteile des Signals und Vergleicher mit Hysterese (36) folgen, welche logische Signale erzeugen, wobei diese paarweise (37, 38) gruppierten logischen Signale, welche als Ursprung die Anordnungen der zwei Sonden (1, 2) haben, durch einen logischen Phasenvergleicher (39) verglichen werden, welcher ein Signal (40) erzeugt, das charakteristisch für die Phasenverschiebung zwischen den beiden von den Sonden gelieferten Signalen ist;

c) eine Anzeigevorrichtung (54, 55, 56), welche die Schnittstelle zwischen der Vorrichtung und dem Operateur bildet.

2. Vorrichtung nach Anspruch 1, **dadurch gekennzeichnet**, daß sie Anordnungen mit zwei auf einer Achse angeordneten Sonden (1, 2) aufweist, denen in zwei oder mehreren Richtungen (19, 20, 21 und 22) eine gleiche Sonde zugeordnet ist.

3. Vorrichtung nach den Ansprüchen 1 und 2, **dadurch gekennzeichnet**, daß sie zwei Anordnungen mit zwei Sonden auf zwei rechtwinklig zueinander verlaufenden Achsen (19 und 20) hat, welche mittels einer Schaltung zwei Phasenverschiebungssignale (25 und 28) und von einem Vektor (29) des Bildes der myokardischen Depolarisation Projektionsbilder in der Ebene der Auflage (7) der Sonde erzeugt und daß sie eine Schnittstelle (43) zum Wiederzusammensetzen des genannten Vektors (29) enthält.

4. Vorrichtung nach Anspruch 1, **dadurch gekennzeichnet**, daß die beiden Sonden (1 und 2) auf einer Nadel (32) zum Bestimmen des Mittelpunktes der Aktivierung angeordnet sind.

5. Vorrichtung nach Anspruch 4, **dadurch gekennzeichnet**, daß die Nadel Mittel (21, 52) zum Beseitigen der Austrittsstelle hat, welche von der Schnittstelle (43) zugelassen und durch den Operateur gesteuert sind.

6. Vorrichtung nach Anspruch 1, **dadurch gekennzeichnet**, daß die Verstärker (34) einen Begrenzer (45) zum Schutz vor Überspannung aufweisen.

7. Vorrichtung nach Anspruch 1, **dadurch gekennzeichnet**, daß sie einen analogen Unterbrecher (41) enthält, welcher die Signale eliminiert, welche von einem Herzschrittmacher stammen, wobei der Unterbrecher von den direkt vom Schrittmacher kommenden elektrischen Impulsen gesteuert ist.

8. Vorrichtung nach Anspruch 1, **dadurch gekennzeichnet**, daß die Anzeigevorrichtung eine Schnittstelle (43) zwischen der Vorrichtung und dem Operateur durch einen seiner physiologischen Sinne, Sehen, Hören, Fühlen aufweist, welches ihm einerseits entweder die Seite und Richtung (29), nach der die myokardische Depolarisation sich ausbreitet oder die gegenüberliegende Seite in gleicher Ausrichtung (29′) anzeigt, wodurch die Verschieberichtung der Sonde erkennbar wird, um die folgende Etappe der Untersuchung durchzuführen, andererseits gegebenenfalls die Lage der Austrittsstelle (12′ oder 12″) unter der Sonde beim Fehlen einer Phasenverschiebung in allen Richtungen anzeigt.

9. Vorrichtung nach der Kombination der vorangehenden Ansprüchen, **dadurch gekennzeichnet**, daß sie einen Rechner (47) zur Signalverarbeitung nach der Verstärkung aufweist und mit peripheren Signal-Abtastschaltungssperren und einem Analog-Digital-Umwandler (46, 46′, 46″ und 46‴) und einer Interface-Einrichtung (48 und 48′) für den Anwender (44) ausgestattet ist.

**Claims**

1. Device intended for the localization of the point of emergence of ventricular tachycardias in cardiological medicine, characterised in that it comprises:

a) an assembly of two sensors (1) and (2), spaced less than 50 millimetres apart and disposed on a

7

rectilinear axis (3);

b) an electronic controlling device, comprising as many amplifiers (34) as there are sensors (1) or (2), each followed by filters (35), intended to select the useful component of the signal, and by hysteresis comparators (36) producing logical signals, and these logical signals, grouped in twos (37) and (38) and originating in the assemblies of two sensors (1) and (2), are compared by a logical phase detector (39) producing a signal (40) which is characteristic of the phase difference between the signals provided by the two sensors; and

c) a signalling device (54, 55, 56) forming the interface between device and operator.

2. Device according to claim 1, characterised in that it comprises assemblies of two sensors (1) and (2) disposed on an axis and associated with the same probe according to two or a plurality of directions (19, 20, 21 and 22).

3. Device according to claims 1 and 2, characterised in that it comprises two assemblies of two sensors, disposed according to two perpendicular axes (19 and 20), producing, by means of a circuit, two unphased signals (25) and (28), images of the projections in the plane of the support (7) of the probe, a vector image (29) of the myocardial depolarisation, and in that it comprises an interface (43) for reconstituting said vector (29).

4. Device according to claim 1, characterised in that the two sensors (1) and (2) are disposed on a needle (32) which localises the centre of activation.

5. Device according to claim 4, characterised in that the needle comprises a means (51) and (52) for destroying the point of emergence authorised by the interface (43) and controlled by the operator.

6. Device according to claim 1, characterised in that the amplifiers (34) comprise a limiter (45) for protection against excess voltage phenomena.

7. Device according to claim 1, characterised in that it comprises an analogical contact breaker (41), eliminating the signals arising from a cardiac stimulator, the contact breaker being controlled by the electrical impulses derived directly from the stimulator.

8. Device according to claim 1, characterised in that the signalling device comprises an interface means (43) between the device and the operator, via the intermediary of one of his/her physiological senses: sight, hearing, touch, indicating to him/her:

- on the one hand, either the sense and the direction (29) in which the myocardial depolarisation spreads, or the opposite sense in the same direction (29′) indicating the orientation of the displacement of the probe in order to achieve the next stage of data sampling.
- on the other hand, should the occasion arise, the localisation of the point of emergence (12′ or 12″) beneath the probe, in the absence of a phase difference in all directions.

9. Device according to the preceding claims taken in their entirely, characterised in that it comprises a computer (47) for processing the signal after amplification and is provided with peripherals for sample-blocking and analog-digital conversion (46), (46′), (46″) and (46‴) and interfacing (48) and (48′) with the user (44).

Fig. 1a

Fig. 1b

Fig. 2

Coupe A-A

Fig. 3a

Fig. 3b

## Fig. 4a

## Fig. 4b

## Fig. 5

## Fig. 6

## Fig. 7

Fig. 8

Fig. 9

Fig. 10

Fig. 11

Fig. 12

Fig. 13

Fig. 14a

Fig. 14b